# EUROPEAN PATENT SPECIFICATION

(11) **EP 2 892 498 B1**
(45) Date of publication and mention of the grant of the patent: **25.10.2017**
(21) Application number: 13759690.4
(22) Date of filing: 02.09.2013
(51) Int. Cl.: A61K 8/02, A61K 8/73, A61K 8/97, A61Q 5/02, A61Q 19/10

(54) **COSMETIC COMPOSITION COMPRISING NATURAL ORGANIC PARTICLES, AND AN ABSORBENT**
KOSMETISCHE ZUSAMMENSETZUNG MIT NATÜRLICHEN ORGANISCHEN PARTIKELN UND EINEM ABSORBER
COMPOSITION COSMÉTIQUE COMPRENANT DES PARTICULES ORGANIQUES NATURELLES ET UN ABSORBANT

(30) Priority: 04.09.2012 FR 1258242; 20.11.2012 US 201261728271 P
(43) Date of publication of application: 15.07.2015
(73) Proprietor: L'OREAL, 75008 Paris (FR)
(72) Inventor: SELLIER, Céline, F-75011 Paris (FR); GABIN, Gérard, F-75009 Paris (FR)
(74) Representative: Casalonga
(86) International application number: PCT/EP2013/068090
(87) International publication number: WO 2014/037305

(56) References cited:
- EP-A1- 0 968 703
- GB-A- 819 709
- JP-A- 2003 342 162
- US-A- 4 508 634
- Anonymous: "Resources, Tools and Basic Information for Engineering and Design of Technical Applications! Densities of Miscellaneous Solids Solids -densities or weights", , 23 May 2014 (2014-05-23), XP055119954, Internet Retrieved from the Internet: URL:http://www.engineeringtoolbox.com/dens ity-solids-d_1265.html [retrieved on 2014-05-26]
- Anonymous: "Agar-Agar : Properties and Specifications", , 1 January 2003 (2003-01-01), XP055119973, Retrieved from the Internet: URL:http://www.agargel.com.br/agar-tec-en. html [retrieved on 2014-05-26]

## Description

The present invention relates to a cosmetic composition comprising at least one type of organic particles, of natural origin, which are insoluble in water and have a density of less than or equal to 0.4, and at least one absorbent. It also relates to the use thereof for cleansing and/or conditioning keratin materials such as the hair, scalp and skin.

Many cosmetic care products are known in the field of hair and body hygiene. For example, the purpose of the corresponding compositions is to impart good cosmetic properties to the hair.

Conventional care products for keratin materials and in particular for the hair and scalp, for example conditioners or masks, are usually in the form of more or less viscous creams. Similarly, cleansing products for keratin materials are usually in the form of more or less viscous liquids.

But these products are difficult to measure out, in particular because they have a tendency to escape between the fingers or to escape from their packaging, which may be very annoying when they come into contact with clothing, for example when travelling.

Furthermore, solid formulations themselves have the drawback of being difficult to divide up.

There is therefore a need to provide care compositions for keratin materials that do not run, that are more compact and mouldable, and that are more natural and economical. The compositions sought must be easy to apply to keratin materials, very light and very soft.

The applicant has surprisingly discovered that a cosmetic composition comprising the combination of organic particles of natural origin, which are insoluble in water and have a density of less than or equal to 0.4, with an absorbent, made it possible to solve the problems mentioned above.

In particular, the compositions according to the invention have a texture and usage qualities that are improved relative to the compositions from the prior art comprising, for example, expanded polymer microspheres. They form solids that are more flexible and more mouldable, less friable, and which are easier to manufacture with conventional processes.

The compositions according to the invention are less likely to break and to escape from the hands.

From a sensory viewpoint, the compositions according to the invention are also better: they provide, in particular, a fresh effect in the hands when they are taken up and in the end give a more natural cosmetic result and cleaner and finer hair.

One subject of the invention is thus a solid cosmetic composition comprising:
- from 5% to 40% by weight, relative to the total weight of the composition, of one or more types of organic particles of natural origin, which are insoluble in water and have a density of less than or equal to 0.4, and
- one or more absorbents.

The composition according to the invention has a quite unusual texture.

The composition preferably has a low density at 25°C, namely of less than 1, preferably ranging from 0.3 to 0.9, better still from 0.5 to 0.9, and is very soft to the touch. It then behaves as a deformable or mouldable solid that is non-tacky and more or less firm.

The composition also has a fresh feel during application.

The expression "fresh feel" is understood for the purposes of the present invention to mean a feeling of freshness when the formula is held in wet hands.

The solid behaviour of the composition according to the invention allows easy handling of the product and ease of transport. In particular, there is no risk of it escaping from its packaging, in particular when it is transported. Since the texture is highly mouldable, the composition can be gripped easily and does not flow between the fingers. It can be measured out and applied very easily. The composition may be easily divided by hand in order to take up only the necessary amount of product. In particular, this composition may be packaged in the single-unit form and may for example be in the form of small cubes or cartons.

The composition according to the invention is also very comfortable to apply. In particular, no running of the composition is observed, unlike conventional compositions, which carries with it the risk of irritating particularly the face and the eyes. The absence of runs is greatly appreciated in the case of permanent waves and dyeing, and also for shampoos intended for children.

In addition, the composition disintegrates rapidly and easily in contact with a liquid, preferably an aqueous liquid.

The term "disintegration" is understood for the purposes of the present invention to mean disintegration with the aid of a liquid and not disintegration upon touch as is the case for certain makeup compositions of eyeshadow type which can be taken up with a finger or using a brush. This disintegration with the aid of a liquid in fact corresponds to a destructuring of the solid, with solubilization or dispersion of the particles in the liquid.

Another subject of the invention is the use of the cosmetic composition according to the invention for cleansing and/or conditioning keratin materials, such as the hair and scalp, and the skin.

Other subjects, characteristics, aspects and advantages of the invention will become even more clearly apparent on reading the description and examples that follow.

In that which follows and unless otherwise indicated, the limits of a range of values are included within this range, in particular in the expressions "between" and "ranging from ... to ...".

Moreover, the expression "at least one" used in the present description is equivalent to the expression "one or more".

According to the invention, the cosmetic composition comprises:
- one or more types of organic particles of natural origin, which are insoluble in water and have a density of less than or equal to 0.4, and
- one or more absorbents.

The organic particles of natural origin, which are insoluble in water and have a density of less than or equal to 0.4, act as a structuring agent.

These organic particles of natural origin are more economical than synthetic organic particles. Furthermore, they usually exhibit a good level of harmlessness and a good profile with respect to ecotoxicity.

With a view to obtaining a solid composition having a pleasant and soft feel, it is preferable to use particles having a mean particle size of from 1 to 250 µm, preferably from 5 to 200 µm, and more preferably still from 10 to 150 µm, and better still from 50 to 150 µm.

This particle size is relative to the largest dimension of the particles.

In order to give the composition of the invention an aerated and light appearance, particles are used that have a density of less than or equal to 0.4, preferably less than 0.2.

The expression "insoluble in water" is understood to mean particles that have a solubility in water at 25°C of less than or equal to 0.5% by weight, better still of less than or equal to 0.1% by weight.

They may have any shape possible and in particular the shape of beads, fibres, plates or needles, or even a slightly rounded cellular shape.

These particles may be made from various inert materials that do not react chemically with the medium. In particular, these particles do not react with oils, surfactants, water and the various other constituents of the composition such as the active agents.

The particles are advantageously chosen from cork oak bark and from wood originating from the trunk or branches of trees, and more particularly of Quercus suber or of balsa Bombax.

The organic particle(s) of natural origin may represent from 5% to 40% by weight, preferably from 5% to 20% by weight, better still from 6% to 15% by weight, relative to the total weight of the cosmetic composition.

As indicated above, the cosmetic composition according to the invention comprises one or more absorbents.

The expression "absorbent" is understood to mean any compound capable of rapidly trapping a large amount of water. The absorbent is therefore generally a hydrophilic or amphiphilic organic compound.

For the purposes of the present invention, the term "absorbent" is understood to mean any compound having a static water absorption capacity, at ambient temperature (25°C), greater than or equal to 3 times its weight.

Preferably, the absorbents are chosen from compounds having a static water absorption capacity greater than or equal to 5 times their weight, and preferably greater than or equal to 15 times their weight.

The test for measuring said static water absorption capacity is the following: at ambient temperature, the compound to be tested, in an amount of x grams, is placed in a beaker; water is added in an amount of 3x grams. It is left to rest, without stirring, for 1 minute.

If no free water (supernatant water) remains after said minute, the compound can be considered to be an absorbent within the meaning of the invention.

The absorbent makes it possible to obtain a composition in the form of a solid, especially a deformable solid, which readily disintegrates with the aid of a diluent, which is generally hot or cold water but which may also be water with the addition of one or more cosmetically acceptable polar solvents, such as alcohols having from 2 to 20 carbon atoms (isopropanol and ethanol in particular), propylene glycol, or else water with the addition of one or more surfactants. It is also possible to use more complex aqueous media.

Preferably, the absorbent(s) is (are) chosen from modified or unmodified starches, and wood flours.

The starch(es) that can be used in the present invention is (are) more particularly macromolecules in the form of polymers formed from elemental units that are anhydroglucose units. The number of these units and their assembly make it possible to distinguish amylose (linear polymer) and amylopectin (branched polymer). The relative proportions of amylose and of amylopectin, and their degree of polymerization, vary as a function of the plant origin of the starches.

The starch molecules used in the present invention may originate from a plant source such as cereals, tubers, roots, legumes and fruits. Thus, the starch(es) may originate from a plant source chosen from corn, pea, potato, sweet potato, banana, barley, wheat, rice, oat, sago, tapioca and sorghum. The starch is preferably derived from potato.

It is also possible to use the hydrolysates of the starches mentioned above.

Starches are generally in the form of a white powder, which is insoluble in cold water, the elemental particle size of which ranges from 3 to 100 microns.

The starches used in the composition of the invention may be chemically modified via one or more of the following reactions: pregelatinization, oxidation, crosslinking, esterification, heat treatments.

More particularly, these reactions may be performed in the following manner:
- pregelatinization by splitting the starch granules (for example drying and cooking in a drying drum);
- oxidation with strong oxidizing agents, leading to the introduction of carboxyl groups into the starch molecule and to depolymerization of the starch molecule (for example by treating an aqueous starch solution with sodium hypochlorite);
- crosslinking with functional agents capable of reacting with the hydroxyl groups of the starch molecules, which will thus bond together (for example with glyceryl and/or phosphate groups);
- esterification in alkaline medium for the grafting of functional groups, especially C₁-C₆ acyl (acetyl), C₁-C₆ hydroxyalkyl (hydroxyethyl or hydroxypropyl), carboxymethyl or octenylsuccinic groups.

Monostarch phosphates (of the type St-O-PO-(OX)₂), distarch phosphates (of the type St-O-PO-(OX)-O-St) or even tristarch phosphates (of the type St-O-PO-(O-St)₂) or mixtures thereof may especially be obtained by crosslinking with phosphorus compounds.

X especially denotes alkali metals (for example sodium or potassium), alkaline-earth metals (for example calcium or magnesium), ammonium salts, amine salts, for instance those of monoethanolamine, diethanolamine, triethanolamine, 3-amino-1,2-propanediol, or ammonium salts derived from basic amino acids such as lysine, arginine, sarcosine, ornithine or citrulline.

The phosphorus compounds may be, for example, sodium tripolyphosphate, sodium orthophosphate, phosphorus oxychloride or sodium trimetaphosphate.

Distarch phosphates or compounds rich in distarch phosphate will especially be used, for instance the products sold under the references Prejel VA-70-T AGGL (gelatinized hydroxypropyl cassava distarch phosphate), Prejel TK1 (gelatinized cassava distarch phosphate) and Prejel 200 (gelatinized acetyl cassava distarch phosphate) by the company Avebe, or Structure Zea from National Starch (gelatinized hydroxypropyl corn distarch phosphate).

According to the invention, it is also possible to use amphoteric starches, these amphoteric starches containing one or more anionic groups and one or more cationic groups. The anionic and cationic groups may be linked to the same reactive site of the starch molecule or to different reactive sites; they are preferably linked to the same reactive site. The anionic groups may be of carboxylic, phosphate or sulfate type, preferably carboxylic. The cationic groups may be of primary, secondary, tertiary or quaternary amine type.

The amphoteric starches are especially chosen from the compounds having the following formulae: 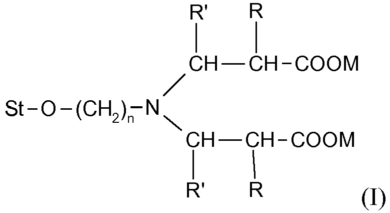 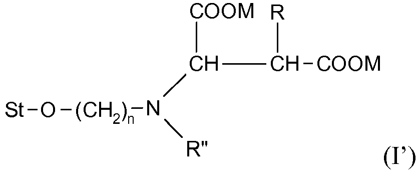 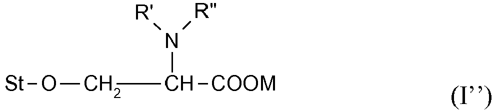 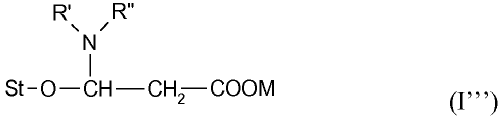 in which formulae:
St-O represents a starch molecule,
R, which may be identical or different, represents a hydrogen atom or a methyl group,
R', which may be identical or different, represents a hydrogen atom, a methyl group or a -COOH group,
n is an integer equal to 2 or 3,
M, which may be identical or different, denotes a hydrogen atom, an alkali metal or alkaline-earth metal such as Na, K, Li or NH₄, a quaternary ammonium or an organic amine,
R" represents a hydrogen atom or an alkyl group containing from 1 to 18 carbon atoms.

These compounds are especially described in patents US 5 455 340 and US 4 017 460.

As amphoteric starches, use is made particularly of the starches of formula (I) or (I'). Use is made more particularly of starches modified with 2-chloroethylaminodipropionic acid, i.e. starches of formula (I) or (I') in which R, R', R" and M represent a hydrogen atom and n is equal to 2. Mention may be made in particular of the potato starch modified with 2-chloroethylaminodipropionic acid neutralized with sodium hydroxide, sold under the reference Structure Solanace by the company National Starch.

The expression "O-carboxymethyl starch" denotes a starch which has been modified by substitution, in free hydroxyl groups, of a hydrogen by a -CH₂COOH carboxymethyl group. It may be present as is, or in the form of a salt, for example an alkali metal salt.

O-carboxylmethyl starches may be prepared, for example, by reacting a starch with monochloroacetic acid, or an alkali metal salt of monochloroacetic acid (for example the sodium salt).

Preferably, use is made of an O-carboxylmethyl starch that is in the form of an alkali metal salt, and more preferably in the form of a sodium salt.

Preferably, the O-carboxylmethyl starch is prepared from starch derived from potato.

The O-carboxylmethyl starch may also be completely or partly crosslinked. Preferably, it is partially crosslinked. The crosslinking of the starch may be carried out, for example, by heating the starch, or by reacting it with crosslinking agents such as phosphates or glycerol.

More preferably still, the O-carboxylmethyl starch is a sodium salt of partially crosslinked O-carboxylmethyl starch, in particular potato starch. Such a product is sold, for example, under the name Primojel by the company Avebe.

The wood flours that can be used as an absorbent in the composition according to the invention may especially be spruce flour or beech flour, preferably spruce flour.

Preferably, the mean particle size of the wood flours is less than 250 µm.

Such products are especially sold by the Société Parisienne des Sciures under the name T140 (spruce flour) or H160/0 (beech flour).

Preferably, the absorbent(s) present in the composition according to the invention is or are chosen from modified or unmodified starches, more particularly modified starches, and better still from modified starches, in particular of potato, such as O-carboxylmethyl starches, in particular of potato.

The absorbent(s) is (are) generally present in an amount ranging from 2% to 20% by weight, preferably from 2% to 10% by weight and better still from 2% to 6% by weight, relative to the total weight of the composition.

In one preferred embodiment, the weight ratio of the type(s) of organic particles, of natural origin, which are insoluble in water and have a density of less than or equal to 0.4, to the absorbent(s) varies from 0.1 to 20, preferably from 0.5 to 15, better still from 1 to 10.

The composition according to the invention preferably comprises water, or a mixture of water and one or more organic solvents, for instance ethanol, propylene glycol, butylene glycol, isopropanol, glycol ethers such as the (C₁-C₄)alkyl ethers of mono-, di- or tripropylene glycol, or of mono-, di- or triethylene glycol, dipropylene glycol, diethylene glycol and mixtures thereof.

In one preferred embodiment, the composition comprises water and does not contain organic solvents.

The composition according to the invention may also comprise one or more surfactants. The surfactant(s) may be chosen from anionic, nonionic, cationic, amphoteric or zwiterrionic surfactants. Preferably, the composition according to the invention comprises at least one anionic or cationic surfactant or a mixture thereof.

In one embodiment of the invention, the composition comprises one or more anionic surfactants.

The term "anionic surfactant" means a surfactant comprising, as ionic or ionizable groups, only anionic groups. These anionic groups are preferably chosen from the following groups: -CO₂H, -CO₂⁻, -SO₃H, -SO₃⁻, -OSO₃H, -OSO₃⁻, -H₂PO₃, -HPO₃⁻, -PO₃²⁻, -H₂PO₂, -HPO₂⁻, -PO₂²⁻, -POH, -PO⁻.

As examples of anionic surfactants that may be used in the composition according to the invention, mention may be made of alkyl sulfates, alkyl ether sulfates, alkylamido ether sulfates, alkylaryl polyether sulfates, monoglyceride sulfates, alkyl sulfonates, alkylamide sulfonates, alkylaryl sulfonates, α-olefin sulfonates, paraffin sulfonates, alkyl sulfosuccinates, alkylether sulfosuccinates, alkylamide sulfosuccinates, alkyl sulfoacetates, acyl sarcosinates, acyl glutamates, alkyl sulfosuccinamates, acyl isethionates and N-acyl taurates, salts of alkyl monoesters of polyglycoside-polycarboxylic acids, acyl lactylates, D-galactoside uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds comprising from 6 to 24 carbon atoms and the aryl group denoting a phenyl group.

These compounds can be oxyethylenated and then preferably comprise from 1 to 50 ethylene oxide units.

The salts of C₆-C₂₄ alkyl monoesters of polyglycoside-polycarboxylic acids can be chosen from C₆-C₂₄ alkyl polyglycoside-citrates, C₆-C₂₄ alkyl polyglycoside-tartrates and C₆-C₂₄ alkyl polyglycoside-sulfo succinates.

When the anionic surfactant(s) is (are) in the salt form, it (they) can be chosen from the alkali metal salts, such as the sodium or potassium salt and preferably the sodium salt, the ammonium salts, the amine salts and in particular the amino alcohol salts, or the alkaline-earth metal salts, such as the magnesium salt.

Mention may in particular be made, as examples of amino alcohol salts, of mono-, di- and triethanolamine salts, mono-, di- or triisopropanolamine salts, 2-amino-2-methyl-1-propanol salts, 2-amino-2-methyl-1,3-propanediol salts and tris(hydroxymethyl)aminomethane salts.

Use is preferably made of alkali metal or alkaline earth metal salts, in particular sodium or magnesium salts.

The anionic surfactants that are optionally present may be mild anionic surfactants, i.e. anionic surfactants without a sulfate function.

As regards the mild anionic surfactants, mention may be made in particular of the following compounds and salts thereof, and also mixtures thereof:
polyoxyalkylenated alkyl ether carboxylic acids,
polyoxyalkylenated alkylaryl ether carboxylic acids,
polyoxyalkylenated alkylamido ether carboxylic acids, in particular those comprising from 2 to 50 ethylene oxide groups,
alkyl-D-galactoside uronic acids,
acyl sarcosinates, acyl glutamates, and
alkyl polyglycoside carboxylic esters.

Use may very particularly be made of polyoxyalkylenated alkyl ether carboxylic acids, such as, for example, lauryl ether carboxylic acid (4.5 EO), for example sold under the name Akypo RLM 45 CA from Kao.

The particularly preferred anionic surfactants are chosen from sulfate and sulfonate anionic surfactants and mixtures thereof, better still from (C₆₋₂₄ alkyl) sulfate salts and (C₆₋₂₄ alkyl) ether sulfate salts, and mixtures thereof.

When an anionic surfactant or anionic surfactants is or are present in the composition according to the invention, the amount thereof preferably ranges from 1% to 40% by weight, better still from 4% to 30% by weight and more preferably still from 8% to 20% by weight, relative to the total weight of the composition.

In another embodiment, the composition comprises one or more cationic surfactants.

The cationic surfactant(s) that can be used in the composition according to the invention comprise, for example, salts of optionally polyoxyalkylenated primary, secondary or tertiary fatty amines, quaternary ammonium salts, and mixtures thereof.

Examples of quaternary ammonium salts that may in particular be mentioned include:
- those corresponding to the general formula (II) below: 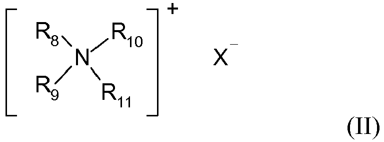
in which the groups R₈ to R₁₁, which may be identical or different, represent a linear or branched aliphatic group comprising from 1 to 30 carbon atoms or an aromatic group such as an aryl or alkylaryl group, at least one of the groups R₈ to R₁₁ comprising from 8 to 30 carbon atoms and preferably from 12 to 24 carbon atoms. The aliphatic groups may comprise heteroatoms especially such as oxygen, nitrogen, sulfur and halogens.

The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl and C₁-C₃₀ hydroxyalkyl groups, X- is an anion chosen from the group of halides, phosphates, acetates, lactates, (C₁-C₄)alkyl sulfates, and (C₁-C₄)alkyl or (C₁-C₄)alkylaryl sulfonates.

Among the quaternary ammonium salts of formula (II), preference is firstly given to tetraalkylammonium chlorides, for instance dialkyldimethylammonium or alkyltrimethylammonium chlorides in which the alkyl group comprises approximately from 12 to 22 carbon atoms, in particular behenyltrimethylammonium, distearyldimethyl-ammonium, cetyltrimethylammonium, dicetyldimethylammonium and benzyldimethylstearylammonium chlorides, or else, secondly, to palmitylamidopropyltrimethylammonium chloride or stearamidopropyl-dimethyl(myristyl acetate)ammonium chloride, which is sold under the name Ceraphyl® 70 by the company Van Dyk.
- quaternary ammonium salts of imidazoline, for instance those of formula (III) below: 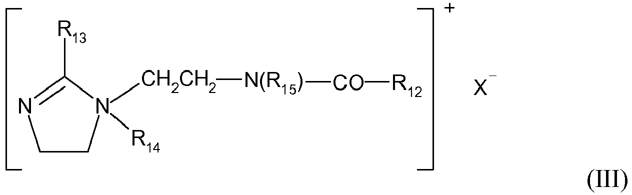 in which R₁₂ represents an alkenyl or alkyl group containing from 8 to 30 carbon atoms, for example tallow fatty acid derivatives, R₁₃ represents a hydrogen atom, a C₁-C₄ alkyl group or an alkenyl or alkyl group containing from 8 to 30 carbon atoms, R₁₄ represents a C₁-C₄ alkyl group, R₁₅ represents a hydrogen atom or a C₁-C₄ alkyl group, and X⁻ is an anion chosen from the group of halides, phosphates, acetates, lactates, (C₁-C₄)alkyl sulfates, and (C₁-C₄)alkyl or (C₁-C₄)alkylaryl sulfonates. R₁₂ and R₁₃ preferably denote a mixture of alkenyl or alkyl groups containing from 12 to 21 carbon atoms, for example tallow fatty acid derivatives, R₁₄ denotes a methyl group, and R₁₅ denotes a hydrogen atom. Such a product is sold, for example, under the name Varisoft W 575 PG N by the company Evonik Goldschmidt;
- quaternary diammonium or triammonium salts, particularly of formula (IV) below: 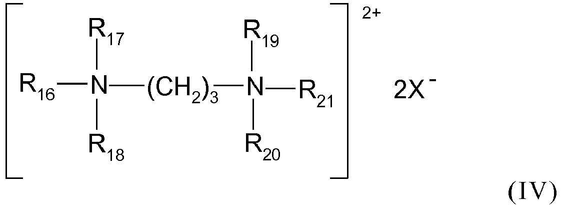 in which R₁₆ denotes an alkyl group containing approximately from 16 to 30 carbon atoms, which is optionally hydroxylated and/or interrupted with one or more oxygen atoms; R₁₇ is chosen from hydrogen, an alkyl group containing from 1 to 4 carbon atoms or a group -(CH₂)₃-N⁺(R₁₆ₐ)(R₁₇ₐ)(R₁₈ₐ); R₁₆ₐ, R₁₇ₐ, R₁₈ₐ, R₁₈, R₁₉, R₂₀ and R₂₁, which may be identical or different, are chosen from hydrogen and an alkyl group containing from 1 to 4 carbon atoms, and X- is an anion chosen from the group of halides, acetates, phosphates, nitrates, (C₁-C₄)alkyl sulfates, and (C₁-C₄)alkyl or (C₁-C₄)alkylaryl sulfonates, in particular methyl sulfate and ethyl sulfate. Such compounds are, for example, Finquat CT-P, sold by the company Innospec Active Chemicals (Quaternium 89), and Condicare CT sold by the company Innospec Active Chemicals (Quaternium 75);
- quaternary ammonium salts containing one or more ester functions, such as those of formula (V) below: 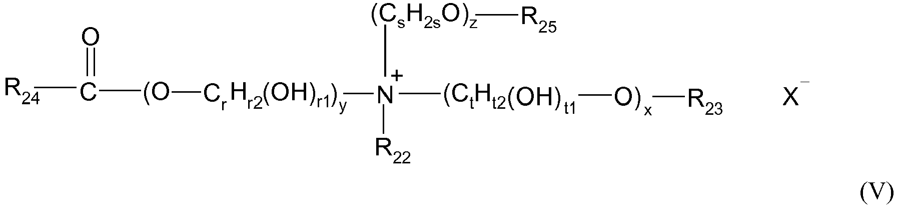 in which:
   R₂₂ is chosen from C₁-C₆ alkyl groups and C₁-C₆ hydroxyalkyl or C₁-C₆ dihydroxyalkyl groups;
   R₂₃ is chosen from:
      - the group 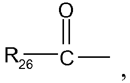
      - linear or branched, saturated or unsaturated C₁-C₂₂ hydrocarbon groups R₂₇,
      - a hydrogen atom,
   R₂₅ is chosen from:
      - the group 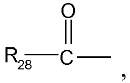
      - linear or branched, saturated or unsaturated C₁-C₆ hydrocarbon groups R₂₉,
      - a hydrogen atom,
   R₂₄, R₂₆ and R₂₈, which may be identical or different, are chosen from linear or branched, saturated or unsaturated C₇-C₂₁ hydrocarbon groups;
   r, s and t, which may be identical or different, are integers ranging from 2 to 6;
   r1 and t1, which may be identical or different, are equal to 0 or 1,
   r2+r1=2r and t1+t2=2t,
   y is an integer ranging from 1 to 10;
   x and z, which may be identical or different, are integers ranging from 0 to 10;
   X⁻ is a simple or complex, organic or inorganic anion;
with the proviso that the sum x + y + z is from 1 to 15, that when x is 0 then R₂₃ denotes R₂₇, and that when z is 0 then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is an R₂₇ hydrocarbon group, it may be long and may have from 12 to 22 carbon atoms, or may be short and may have from 1 to 3 carbon atoms.

When R₂₅ is an R₂₉ hydrocarbon group, it preferably has 1 to 3 carbon atoms.

Advantageously, R₂₄, R₂₆ and R₂₈, which are identical or different, are chosen from linear or branched, saturated or unsaturated C₁₁-C₂₁ hydrocarbon groups, and more particularly from linear or branched, saturated or unsaturated C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and more particularly still are equal to 2.

The anion X⁻ is preferably a halide, preferably chloride, bromide or iodide, a (C₁-C₄)alkyl sulfate, (C₁-C₄)alkyl sulfonate or (C₁-C₄)alkylaryl sulfonate. However, it is possible to use methanesulfonate, phosphate, nitrate, tosylate, an anion derived from an organic acid, such as acetate or lactate, or any other anion that is compatible with the ammonium comprising an ester function.

The anion X⁻ is more particularly still chloride, methyl sulfate or ethyl sulfate.

Use is made more particularly in the composition according to the invention of the ammonium salts of formula (V) in which:
- R₂₂ denotes a methyl or ethyl group,
- x and y are equal to 1;
- z is equal to 0 or 1;
- r, s and t are equal to 2;
- R₂₃ is chosen from:
- the group 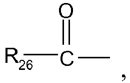
   - methyl, ethyl or C₁₄-C₂₂ hydrocarbon groups,
   - a hydrogen atom;
- R₂₅ is chosen from:
   - the group 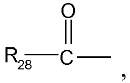
   - a hydrogen atom;
- R₂₄, R₂₆ and R₂₈, which may be identical or different, are chosen from linear or branched, saturated or unsaturated C₁₃-C₁₇ hydrocarbon groups, and preferably from linear or branched, saturated or unsaturated C₁₃-C₁₇ alkyl and alkenyl groups.

The hydrocarbon groups are advantageously linear.

Among the compounds of formula (V), examples that may be mentioned include salts, especially the chloride or methyl sulfate of diacyloxyethyldimethylammonium, diacyloxyethylhydroxyethylmethyl-ammonium, monoacyloxyethyldihydroxyethylmethylammonium, triacyloxyethylmethylammonium or monoacyloxyethylhydroxyethyl-dimethylammonium, and mixtures thereof. The acyl groups preferably contain from 14 to 18 carbon atoms and originate more particularly from a plant oil, such as palm oil or sunflower oil. When the compound contains several acyl groups, these groups may be identical or different.

Mention may be made more particularly of distearoylethylhydroxyethylmethylammonium methosulfate, dipalmitoylethylhydroxyethylammonium methosulfate or distearoylethyl-hydroxyethylammonium methosulfate.

These products are obtained, for example, by direct esterification of triethanolamine, triisopropanolamine, an alkyldiethanolamine or an alkyldiisopropanolamine, which are optionally oxyalkylenated, with fatty acids or with mixtures of fatty acids of plant or animal origin, or by transesterification of the methyl esters thereof. This esterification is followed by a quaternization by means of an alkylating agent such as an alkyl halide, preferably a methyl or ethyl halide, a dialkyl sulfate, preferably a methyl or ethyl sulfate, methyl methanesulfonate, methyl para-toluenesulfonate, glycol chlorohydrin or glycerol chlorohydrin.

The composition according to the invention may contain, for example, a mixture of quaternary ammonium monoester, diester and triester salts with a weight majority of diester salts.

It is also possible to use the ammonium salts containing at least one ester function that are described in patents US-A-4 874 554 and US-A-4 137 180.

Use may be made of behenoylhydroxypropyltrimethylammonium chloride, sold by Kao under the name Quartamin BTC 131.

Preferably, the ammonium salts containing at least one ester function contain two ester functions.

Among the cationic surfactants present in the composition according to the invention, it is more particularly preferred to choose cetyltrimethylammonium, behenyltrimethylammonium or dipalmitoylethylhydroxyethylmethylammonium salts, and mixtures thereof, and more particularly behenyltrimethylammonium chloride, sold for example by the company Clariant under the trade name Genamin KDMP or Genamin BTLF or by the company Evonik Goldschmidt under the name Varisoft BT 85, cetyltrimethylammonium chloride sold, for example, under the trade name Dehyquart A OR by the company Cognis or Quartamin 60 W25 by the company Kao or alternatively Genamin CTAC 25 by the company Clariant, dipalmitoylethylhydroxyethylmethylammonium methosulfate, such as the commercial product Dehyquart F 30 sold by the company Cognis, and mixtures thereof.

Preferably, the cationic surfactants are chosen from the compounds of formula (II) or (V).

When a cationic surfactant or cationic surfactants is or are present in the composition according to the invention, the amount thereof preferably ranges from 0.1% to 20% by weight, better still from 0.2% to 10% by weight and more preferably still from 0.2% to 5% by weight, relative to the total weight of the composition.

The composition according to the invention may additionally comprise any additive capable of being used in the field of application under consideration, such as for example fragrances, UV screening agents, preservatives, antioxidants, pH regulators, sequesterants, free-radical scavengers, moisturisers, reducing agents, conditioning agents other than cationic surfactants, such as esters, silicones and vitamins.

The composition according to the invention may be a shampoo, pre-shampoo, conditioner, hair shaping, permanent waving or hair straightening composition, a dyeing or bleaching composition, a hair conditioning composition or a foaming composition for the skin (body and/or face).

The composition according to the invention is generally rinsed out.

The composition according to the invention may be in the form of a wand, pencil, stick, cake, paste or even cream.

Preferably, the compositions of the invention are deformable solids.

The compositions of the invention may be prepared by conventional processes, in a vessel or by extrusion, preferably by extrusion.

Another subject of the invention is the use of the composition as defined previously for cleansing and/or conditioning keratin materials, such as the hair, the scalp and the skin.

The invention is illustrated by the following examples.

### EXAMPLES

### Examples 1-2

Compositions according to the invention are prepared from the ingredients indicated in Table 1 below. The amounts indicated are expressed as % by weight of raw material relative to the total weight of the composition.

**Table 1**

| | Example 1 | Example 2 |
|---|---|---|
| Cork (*Quercus suber L*.) powder (particle size < 250 µm) | 12.6 | - |
| Balsa (*Ochroma lagopus*) powder | - | 10 |
| Crosslinked sodium carboxymethyl (potato) starch | 4.15 | 4.3 |
| Sodium lauryl ether sulfate (2.2 mol of ethylene oxide) as an aqueous solution | 19 (13.3 AM) | 19 (13.3 AM) |
| Sodium lauryl sulfate powder | 4.15 | 4.3 |
| Sodium benzoate | 0.5 | 0.5 |
| Salicylic acid powder | 0.2 | 0.2 |
| Citric acid·1 H₂O | 0.2 | 0.2 |
| Fragrance | 0.6 | 0.6 |
| Deionized water qs | 100 | 100 |

| | | |
|---|---|---|
| AM: active material | | |

The ingredients are mixed using an extruder and compositions are obtained in the form of a slightly tacky mouldable paste.

Due to the nature of the starting ingredients, these two compositions have a reduced cost compared to the prior art compositions, prepared with synthetic organic water-insoluble particles (for example Expancel 551 from Akzo Nobel).

They have a fresh feel during application and impart a feeling of freshness and cleanliness during the use thereof.

Moreover, they exhibit better disintegration during the application.

Applied to the scalp and hair, improved cleansing is observed, in particular by the mechanical effect provided by the natural organic particles, and more particularly with composition 2.

### Comparative example 1

The following compositions are prepared from the ingredients indicated in Tables 2 and 3 below. The amounts indicated are expressed as % by weight of raw material relative to the total weight of the composition.

**Table 2**

| | | Composition A1 (Invention) | Composition A2 (Comparative) |
|---|---|---|---|
| Absorbent | Crosslinked carboxymethyl potato starch | 27 | 27 |
| | Spruce wood flour | 22 | 22 |
| Structuring agent | Ground cork | 5 | - |
| | Expanded acrylonitrile/methyl methacrylate copolymer microspheres (Expancel) | - | 5 |
| Liquid phase | Propylene glycol | 16 | 16 |
| | Cetyltrimethylammonium chloride | 4 | 4 |
| | Dimethyldiallylammonium chloride/acrylic acid copolymer | 2.7 | 2.7 |
| | Water qs | 100% | 100% |

Compositions A1 and A2 are manufactured by extrusion.

It is observed that composition A1 according to the invention is less friable than comparative composition A2.

**Table 3**

| | | Composition B1 (Invention) | Composition B2 (Comparative) |
|---|---|---|---|
| Absorbent | Crosslinked carboxymethyl potato starch | 5 | 5 |
| | Spruce wood flour | 22 | 22 |
| Structuring agent | Ground cork | 5 | - |
| | Expanded acrylonitrile/methyl methacrylate copolymer microspheres (Expancel) | - | 5 |
| Liquid phase | Propylene glycol | 16 | 16 |
| | Cetyltrimethylammonium chloride | 16 | 16 |
| | Dimethyldiallylammonium chloride/acrylic acid copolymer | 2.7 | 2.7 |
| | Water qs | 100% | 100% |

Compositions B1 and B2 are manufactured conventionally, in a production vessel, with a powder mixer.

It is observed that comparative composition B2 is not homogenous at the end of manufacture and is friable. The composition B1 according to the invention is solid and mouldable.

### Comparative Example 2

The following compositions are prepared from the ingredients indicated in Table 4 below. The amounts indicated are expressed as % by weight of active material with respect to the total weight of the composition.

**Table 4**

| | | Composition C1 (Invention) | Composition C2 (Comparative) |
|---|---|---|---|
| Absorbent | Crosslinked carboxymethyl potato starch | 4.15 | 4.15 |
| Structuring agent | Ground cork | 12.6 | - |
| | Expanded acrylonitrile/methyl methacrylate copolymer microspheres (Expancel) | - | 5 |
| Liquid phase | Sodium lauryl ether sulfate as an aqueous solution | 13.3 | 13.3 |
| | Water qs | 100% | 100% |

A comparative composition C', similar to composition C1, is also prepared by replacing the 12.6% by weight of cork oak flour with 12.6% by weight of Expancel. An inhomogeneous formula is obtained, in powder form, that is not compact and which is not possible to test.

The comparative composition C2 comprises the same volume of structuring agent as composition C1 of the invention. It is observed that the comparative composition C2 is not mouldable whereas composition C1 according to the invention is solid and mouldable.

The hair is cleansed with these two compositions, by applying each composition to half a head, like with a standard shampoo. After rinsing and drying, it is observed that the head treated with composition C1 according to the invention is lighter, cleaner, finer, and appears more natural. The drying is also faster.

## Claims

1. Solid cosmetic composition comprising:
- from 5% to 40% by weight, relative to the total weight of the composition, of one or more types of organic particles of natural origin, which are insoluble in water and have a density of less than or equal to 0.4, and
- one or more absorbents which is a compound having a static water absorption capacity, at ambient temperature (25°C), greater than or equal to 3 times its weight.

2. Composition according to Claim 1, **characterized in that** the particles have a mean particle size of from 1 to 250 µm, preferably from 5 to 200 µm, and more preferably still from 10 to 150 µm, and better still from 50 to 150 µm.

3. Composition according to Claim 1 or 2, **characterized in that** the particles are chosen from cork oak bark and from wood originating from the trunk or branches of trees, better still of Quercus suber or of balsa Bombax.

4. Composition according to any one of the preceding claims, **characterized in that** the particles are present in an amount ranging from 5% to 20% by weight, better still from 6% to 15% by weight, relative to the total weight of the composition.

5. Composition according to any one of the preceding claims, **characterized in that** the absorbents have a static water absorption capacity, at ambient temperature (25°C), of greater than or equal to 5 times their weight and preferably greater than or equal to 15 times their weight.

6. Composition according to any one of the preceding claims, **characterized in that** the absorbent(s) is (are) chosen from modified or unmodified starches, and wood flours.

7. Composition according to Claim 6, **characterized in that** the absorbent(s) is (are) chosen from modified starches, and more particularly O-carboxymethyl starches.

8. Composition according to any one of the preceding claims, **characterized in that** the absorbent(s) is (are) present in an amount ranging from 2% to 20% by weight, preferably from 2% to 10% by weight and better still from 2% to 6% by weight, relative to the total weight of the composition.

9. Composition according to any one of the preceding claims, **characterized in that** the weight ratio of the type(s) of organic particles, of natural origin, which are insoluble in water and have a density of less than or equal to 0.4, to the absorbent(s) varies from 0.1 to 20, preferably from 0.5 to 15, better still from 1 to 10.

10. Composition according to any one of the preceding claims, **characterized in that** it contains water.

11. Composition according to any one of the preceding claims, **characterized in that** it comprises one or more anionic surfactants, preferably chosen from (C₆-₂₄ alkyl) sulfate salts and (C₆-₂₄ alkyl) ether sulfate salts, and mixtures thereof.

12. Composition according to Claim 11, **characterized in that** the anionic surfactants(s) is (are) present in an amount ranging from 1% to 40% by weight, better still from 4% to 30% by weight and more preferably still from 8% to 20% by weight, relative to the total weight of the composition.

13. Composition according to any one of the preceding claims, **characterized in that** it comprises one or more cationic surfactants.

14. Composition according to Claim 13, **characterized in that** the cationic surfactants(s) is (are) present in an amount ranging from 0.1% to 20% by weight, better still from 0.2% to 10% by weight and more preferably still from 0.2% to 5% by weight, relative to the total weight of the composition.

15. Composition according to any of the preceding claims, **characterized in that** it is in the form of a deformable solid.

16. Composition according to any one of the preceding claims, **characterized in that** it is prepared by extrusion.

17. Use of the cosmetic composition according to any one of the preceding claims, for cleansing and/or conditioning keratin materials.

## Patentansprüche

1. Feste Kosmetikzusammensetzung, die Folgendes umfasst:
- 5 Gew.-% bis 40 Gew.-% in Bezug auf das Gesamtgewicht der Zusammensetzung an einer oder mehreren Arten von organischen Teilchen natürlichen Ursprungs, die in Wasser unlöslich sind und eine Dichte von 0,4 oder weniger aufweisen, sowie
- ein oder mehrere Absorptionsmittel, bei dem es sich um eine Verbindung mit einem statischen Wasseraufnahmevermögen bei Raumtemperatur (25°C) von 3-mal ihres Gewichts oder mehr handelt.

2. Zusammensetzung nach Anspruch 1, **dadurch gekennzeichnet, dass** die Teilchen eine mittlere Teilchengröße von 1 bis 250 µm, vorzugsweise 5 bis 200 µm, und noch stärker bevorzugt 10 bis 150 µm, noch besser 50 bis 150 µm, aufweisen.

3. Zusammensetzung nach Anspruch 1 oder 2, **dadurch gekennzeichnet, dass** die Teilchen aus Korkeichenrinde und aus Holz, das vom Stamm oder von Ästen von Bäumen, noch besser von Quercus suber oder von Bombax-Balsa, ausgewählt sind.

4. Zusammensetzung nach einem der vorhergehenden Ansprüche, **dadurch gekennzeichnet, dass** die Teilchen in einer Menge von 5 Gew.-% bis 20 Gew.-%, noch besser 6 Gew.-% bis 15 Gew.-%, in Bezug auf das Gesamtgewicht der Zusammensetzung vorliegen.

5. Zusammensetzung nach einem der vorhergehenden Ansprüche, **dadurch gekennzeichnet, dass** die Absorptionsmittel ein statisches Wasseraufnahmevermögen bei Raumtemperatur (25°C) von 5-mal ihrem Gewicht oder mehr und vorzugsweise 15-mal ihrem Gewicht oder mehr aufweisen.

6. Zusammensetzung nach einem der vorhergehenden Ansprüche, **dadurch gekennzeichnet, dass** das/die Absorptionsmittel aus modifizierten oder unmodifizierten Stärken und aus Holzmehlen ausgewählt ist/sind.

7. Zusammensetzung nach Anspruch 6, **dadurch gekennzeichnet, dass** das/die Absorptionsmittel aus modifizierten Stärken, insbesondere O-Carboxymethylstärken, ausgewählt ist/sind.

8. Zusammensetzung nach einem der vorhergehenden Ansprüche, **dadurch gekennzeichnet, dass** das/die Absorptionsmittel in einer Menge von 2 Gew.-% bis 20 Gew.-%, vorzugsweise 2 Gew.-% bis 10 Gew.-%, noch besser 2 Gew.-% bis 6 Gew.-% in Bezug auf das Gesamtgewicht der Zusammensetzung vorliegt/vorliegen.

9. Zusammensetzung nach einem der vorhergehenden Ansprüche, **dadurch gekennzeichnet, dass** das Gewichtsverhältnis der Art(en) der organischen Teilchen natürlichen Ursprungs, die wasserunlöslich sind und eine Dichte von 0,4 oder weniger aufweisen, zu dem/den Absorptionsmittel(n) im Bereich von 0,1 bis 20, vorzugsweise 0,5 bis 15, noch besser 1 bis 10, beträgt.

10. Zusammensetzung nach einem der vorhergehenden Ansprüche, **dadurch gekennzeichnet, dass** sie Wasser enthält.

11. Zusammensetzung nach einem der vorhergehenden Ansprüche, **dadurch gekennzeichnet, dass** sie ein oder mehrere anorganische Tenside, vorzugsweise ausgewählt aus (C₆₋₂₄-Alkyl) sulfatsalzen und (C₆₋₂₄-Alkyl)ethersulfatsalzen und Mischungen davon umfasst.

12. Zusammensetzung nach Anspruch 11, **dadurch gekennzeichnet, dass** das anionische Tensid/die anionischen Tenside in einer Menge im Bereich von 1 Gew.-% bis 40 Gew.-%, noch besser 4 Gew.-% bis 30 Gew.-% und noch stärker bevorzugt 8 Gew.-% bis 20 Gew.-% in Bezug auf das Gesamtgewicht der Zusammensetzung vorliegt/vorliegen.

13. Zusammensetzung nach einem der vorhergehenden Ansprüche, **dadurch gekennzeichnet, dass** sie ein oder mehrere kationische Tenside umfasst.

14. Zusammensetzung nach Anspruch 13, **dadurch gekennzeichnet, dass** das kationische Tensid/die kationischen Tenside in einer Menge im Bereich von 0,1 Gew.-% bis 20 Gew.-%, noch besser 0,2 Gew.-% bis 10 Gew.-% und noch stärker bevorzugt 0,2 Gew.-% bis 5 Gew.-% in Bezug auf das Gesamtgewicht der Zusammensetzung vorliegt/vorliegen.

15. Zusammensetzung nach einem der vorhergehenden Ansprüche, **dadurch gekennzeichnet, dass** sie in Form eines verformbaren Feststoffs vorliegt.

16. Zusammensetzung nach einem der vorhergehenden Ansprüche, **dadurch gekennzeichnet, dass** sie mittels Extrusion hergestellt wird.

17. Verwendung der Kosmetikzusammensetzung nach einem der vorhergehenden Ansprüche zum Reinigen und/oder Konditionieren von Keratinsubstanzen.

## Revendications

1. Composition cosmétique solide comprenant :
- de 5 à 40 % en poids, par rapport au poids total de la composition, d'un ou plusieurs types de particules organiques, d'origine naturelle, insolubles dans l'eau, et de densité inférieure ou égale à 0,4, et
- un ou plusieurs agents absorbants qui est un composé ayant une capacité statique d'absorption d'eau, à température ambiante (25°C), supérieure ou égale à 3 fois son poids.

2. Composition selon la revendication 1, **caractérisée en ce que** les particules présentent une granulométrie moyenne de 1 à 250 µm, de préférence de 5 à 200 µm, et encore plus préférentiellement de 10 à 150 µm, et encore mieux de 50 à 150 µm.

3. Composition selon la revendication 1 ou 2, **caractérisée en ce que** les particules sont choisies parmi l'écorce de chêne liège et du bois provenant de tronc ou de branches d'arbres, mieux de quercus suber ou de balsa bombax.

4. Composition selon l'une quelconque des revendications précédentes, **caractérisée en ce que** les particules sont présentes en une quantité allant de 5 à 20 % en poids, mieux de 6 à 15 % en poids par rapport au poids total de la composition.

5. Composition selon l'une quelconque des revendications précédentes, **caractérisée en ce que** les agents absorbants ont une capacité statique d'absorption d'eau, à température ambiante (25°C), supérieure ou égale à 5 fois leur poids, et préférentiellement supérieure ou égale à 15 fois leur poids.

6. Composition selon l'une quelconque des revendications précédentes, **caractérisée en ce que** le ou les agents absorbants sont choisis parmi les amidons modifiés ou non, et les farines de bois.

7. Composition selon la revendication 6, **caractérisée en ce que** le ou les agents absorbants sont choisis parmi les amidons modifiés, et plus particulièrement les amidons O-carboxyméthylés.

8. Composition selon l'une quelconque des revendications précédentes, **caractérisée en ce que** le ou les agents absorbants sont présents en une quantité allant de 2 à 20% en poids, de préférence de 2 à 10%, mieux de 2 à 6% en poids par rapport au poids total de la composition.

9. Composition selon l'une quelconque des revendications précédentes, **caractérisée en ce que** le rapport pondéral du ou des type(s) de particules organiques, d'origine naturelle, insolubles dans l'eau, et de densité inférieure ou égale à 0,4, sur l'agent ou les agents absorbant(s) varie de 0,1 à 20, de préférence de 0,5 à, 15, mieux de 1 à 10.

10. Composition selon l'une quelconque des revendications précédentes, **caractérisée en ce qu'**elle contient de l'eau.

11. Composition selon l'une quelconque des revendications précédentes, **caractérisée en ce qu'**elle comprend un ou plusieurs agents tensioactifs anioniques, de préférence choisis parmi les sels d'(alkyl en C₆₋₂₄)sulfate et d'(alkyl en C₆₋₂₄)éther sulfate, et leurs mélanges.

12. Composition selon la revendication 11, **caractérisée en ce que** le ou les agents tensioactifs anioniques sont présents en une quantité allant de 1 à 40 % en poids, mieux de 4 à 30 % en poids, et encore plus préférentiellement de 8 à 20 % en poids, par rapport au poids total de la composition.

13. Composition selon l'une quelconque des revendications précédentes, **caractérisée en ce qu'**elle comprend un ou plusieurs agents tensioactifs cationiques.

14. Composition selon la revendication 13, **caractérisée en ce que** le ou les agents tensioactifs cationiques sont présents dans une quantité allant de 0,1 à 20 % en poids, mieux de 0,2 à 10 % en poids, et encore plus préférentiellement de 0,2 à 5 % en poids, par rapport au poids total de la composition.

15. Composition selon l'une quelconque des revendications précédentes, **caractérisée en ce qu'**elle se présente sous forme d'un solide déformable.

16. Composition selon l'une quelconque des revendications précédentes, **caractérisée en ce qu'**elle est préparée par extrusion.

17. Utilisation de la composition cosmétique selon l'une quelconque des revendications précédentes pour le nettoyage et/ou le conditionnement des matières kératiniques.
